# EUROPEAN PATENT APPLICATION

(11) **EP 2 845 591 A1**
(43) Date of publication of application: **11.03.2015**
(21) Application number: 13182970.7
(22) Date of filing: 04.09.2013
(51) Int. Cl.: A61K 31/19, A61P 17/12, A61K 9/00, A61K 36/00, A61K 31/23, A61K 31/60, A61K 31/194, A61K 31/17

(54) **Use of trifluoroacetic acid as keratolytic agent to treat hyperkeratotic skin lesions**

(71) Applicant: POLICHEM S.A., 1526 Luxembourg (LU)
(72) Inventor: Mailland, Federico, 6900 LUGANO (CH); Iob, Giuliana, 6953 LUGAGGIA (CH)
(74) Representative: Minoja, Fabrizio

(57) **Abstract**

The present invention is directed to the use of Trifluoroacetic acid and its physiologically acceptable salts as keratolytic agent to treat skin lesions characterized by the production of excessive skin by the epidermis.

## Description

The present invention relates to Trifluoroacetic acid and its physiologically acceptable salts for use to treat hyperkeratoses.

### BACKGROUND OF THE INVENTION

A number of skin diseases and conditions, different in pathogenic causes, have in common thickening of the external layer of the epidermis, also called stratum corneum, often associated with a qualitative abnormality of the keratin (the constituent protein of the stratum corneum), and also usually accompanied by an increase in the underlying granular layer. They include actinic (solar) keratosis, common warts, genital warts, follicular hyperkeratinization and acne, plantar hyperkeratosis, and the like.

Actinic keratosis (also called "solar keratosis" and "senile keratosis") is a condition of thick, scaly or crusty patches of skin (Fitzpatrick's Dermatology in General Medicine, McGraw-Hill 2003, pp. 720-721). It is more common in fair-skinned people and it is associated with those who are frequently exposed to the sun, as it is usually accompanied by solar damage. Progressive development of these lesions occurs when skin is exposed to the sun constantly and thick, scaly or crusty areas appear. The scaly or crusty portion is dry and rough. The lesions start out as flat scaly areas and later grow into a tough, wart-like area. An actinic keratosis site commonly ranges between 2 and 6 millimeters in size, and may be dark or light, tan, pink, red, a combination of all these or have the same pigment as the surrounding skin. The lesion may appear on any sun-exposed area, such as the face, ears, neck, scalp, chest, backs of hands, forearms, or lips.

A common wart (or verruca vulgaris, verruca plana) is generally a small, rough growth, typically on a human's hands or feet but often other locations, that can resemble a cauliflower or a solid blister. They are caused by a viral infection, specifically by one of the many types of human papillomavirus (HPV) (Fitzpatrick's Dermatology in General Medicine, McGraw-Hill 2003, pp. 2119-2125). There are many varieties of warts, the most common considered to be mostly harmless. It is possible to get warts from others; they are contagious and usually enter the body in an area of broken skin. They can last for years and can recur after chemical or surgical removal.

Genital warts (or condylomata acuminata, venereal warts, anal warts and anogenital warts) are symptoms of a sexually transmitted disease caused by some sub-types of human papillomavirus (HPV). It is spread through direct skin-to-skin contact during oral, genital or anal sex with an infected partner. Warts are the most easily recognized symptom of genital HPV infection, and types 6 and 11 are responsible for 90% of genital warts cases. The viral particles are able to penetrate the skin and mucosal surfaces through microscopic abrasions in the genital area, which occur during sexual activity. Genital warts often occur in clusters and can be very tiny or can spread into large masses in the genital area. In other cases they look like small stalks. In women they occur either outside or inside the vagina, on cervix or around (or inside) the anus. They are approximately similarly prevalent in men but the symptoms may be less obvious. When present, they usually are seen on the tip of the penis. Rarely, genital warts also can develop in the mouth or throat of a person who has had oral sex with an infected person.

Follicular hyperkeratinization is a skin condition characterized by excessive development of keratin in sebaceous follicles, resulting in rough, cone-shaped, elevated papules. The openings are often closed with a white plug of encrusted sebum. This is the initial factor in comedo formation, which is at the basis of acne (Fitzpatrick's Dermatology in General Medicine, McGraw-Hill 2003, pp. 674-675).

Plantar hyperkeratosis is hyperkeratosis of the sole of the foot. It is often painful and requires periodic removal of the dead skin, to provide symptomatic relief.

All the above conditions may be treated surgically, by excision of the skin/mucosal excess, or may benefit of the treatment with keratolytic agents. Among the keratolytic treatments, the mostly spread is salicylic acid. It requires continuous applications and periodic removal of the uppermost skin layers. The treatment is long-lasting and painful. For example, the treatment of a common wart requires at least 12 weeks of applications of salicylic acid products. Genital warts may be treated by podophyllotoxine and imiquimod, but these agents are corrosive and must be applied very carefully only on the skin lesions. Actinic keratosis may benefit of the treatment with diclofenac, but again the treatment is long-lasting, at least 30 days of twice-daily applications.

As a consequence, there is still an important medical need of safe and effective treatment of hyperkeratotic conditions.

Trifluoroacetic acid is a small molecule already known as the terminal metabolite of the systemic anaesthetic agent halothane. TFA is not known in therapy, though its sodium salt (NaTFA) is known to be devoid of significant toxicity and its calcium salt is known to inhibit tumor growth in animal experimental models and exerts *in vitro* cytotoxic activity on solid tumor cells (WO 03/006031). Calcium trifluoroacetate has further demonstrated its in vitro cytotoxic activity against multiple myeloma, chronic and acute myeloid leukemia human cell lines as well as human marrow stem cells (WO 2006/032458). The calcium salt of trifluoroacetic acid also displays a relevant antiangiogenetic activity (Bussolati B. et al. Microvascular Research 2009, 78:272-277) suggesting a potential use for the preparation of medicaments for the treatment of atherosclerotic plaque, rheumatoid arthritis, psoriasis, diabetic retinopathy, rosacea and cheloids (WO 2006/000339).

It has now been surprisingly found that trifluoroacetic acid has an important exfoliating activity, useful for treating conditions where the use of a keratolytic agent is required.

### DESCRIPTION OF THE INVENTION

The invention concerns the use of trifluoroacetic acid, or a physiologically acceptable salt thereof, as keratolytic agent to treat skin or mucosal lesions characterized by the production of excessive skin by the epidermis.

For the purposes of the invention, trifluoroacetic acid, or a physiologically acceptable salt thereof, may be suitably formulated for topical application.

For topical application, trifluoroacetic acid, or a salt thereof, may be in the form of semi-solid or liquid formulations containing trifluoroacetic acid or a salt thereof, together with at least a pharmaceutically acceptable excipient and/or adjuvant; such formulations may be in the form of solutions, emulsions or suspensions, creams, gels, sticks and ointments.

Such semi-solid or liquid formulations may have a w/w concentration in trifluoroacetic acid from 0.1% to 20%, more preferably from 1% to 15%, most preferably from 5% to 10%. They are particularly suitable to treat skin or mucosal thickening lesions by direct application over the lesions.

These pharmaceutical compositions may be prepared according to conventional techniques, may contain pharmaceutically acceptable excipients, adjuvants and/or carriers and may also contain, in combination, one or more active principles with complementary or, in any case, useful activity.

The active agents which may be used in combination with trifluoroacetic acid in the treatment of the present invention include, but are not limited to, keratolytic agents such as dicarboxylic acids, esters or amides, plant extracts and their phytosomes®, emollients, moisturizers and humectants, plasticizers, anti-oxidants; silicone compounds (siloxanes and silanes); film-forming, suspending and viscosity-increasing polymers; anti-microbials. Said active ingredients may be administered together with trifluoroacetic acid (i.e. they may be for instance contained in the same composition as trifluoroacetic acid) or they may be administered separately from or in temporal proximity with trifluoroacetic acid, either by systemic (oral, intravenous, intramuscular) route or by topical route, directly on the skin or mucosal lesions.

Examples of keratolytic agents include alfa-hydroxyacids, beta-hydroxyacids, salycilic acid, glycolic acid, urea, lactic acid, citric acid, malic acid; dicarboxylic acids, esters, or amides such as azelaic acid, potassium azeloyl diglycinate; examples of plant extracts include Serenoa serrulata Fruit Extract, Sesamum indicum Seed oil, Argania spinosa kernel oil, bisabolol, trimethylglycine, Boswellia Serrata (Olibanum) resin extract, Vaccinium myrtillus seed oil, Oenothera biennis oil, Glycyrrhiza glabra root extract, Epilobium fleischeri extract, Avena sativa extract, Aloe vera extract; examples of emollients include heptanoic acid 2,2-dimethyltrimethylene ester, polyoxypropylene alkyl ether, lactic acid dodecyl ester, carbonic acid bis (2-ethylhexyl) ester, essential fatty acids (linoleic and linolenic acids), triglycerides, sterol esters, phytosterols, wax esters, free sterols, free fatty acids, phospholipids, C6-C22 alcohols, n-alkanes, such as paraffinum liquidum, petrolatum, hydrogenated polydecenes; examples of moisturizers and humectants include polyalcohols, glycerin, sorbitol, propanediol, propylene glycol, dipropylene glycol, polyethylene glycols (PEGs, panthenol; NMF (Natural Moisturizing Factor) components, amino acids, pyrrolidone carboxylic acid and their salts, sodium lactate, peptides; examples of plasticizers include C1-C5 alcohols, urea and ureido compounds, such as allantoin; examples of antioxidants include vitamins and their derivatives, tocopherol, ascorbyl palmitate, tocopheryl acetate; examples of silicone compounds are dimethicones, dimethiconols, alkyl dimethicone, alkyl dimethicone copolyols, silicone esters, cyclomethicones, silica, silicone elastomers and resins; examples of film-forming, suspending and viscosity-increasing agents include polymers of natural origin like xanthan gum, cellulose derivatives, chitosan derivatives and of synthetic origin, like povidone, copovidone, acrylic derivatives (e.g. carbomers), polyamide polymers, alkylamide copolymers; examples of anti-microbials include decylene glycol, pentylene glycol, benzyl alcohol, phenetyl alcohol, caprylyl glycol, phenylpropanol, ethylhexylglycerin, salicylic acid.

Examples of the compositions prepared according to the present invention include creams, gels, ointments, solutions, emulsions and suspensions for topical application.

The pharmaceutical compositions and the uses of the present invention will now be more fully described by the following examples.

### EXAMPLE 1

A topical gel formulation, having the following w/w % composition, was prepared:

| | |
|---|---|
| 1. Trifluoroacetic Acid | 0.14% |
| 2. Sodium Trifluoroacetate | 11.10% |
| 3. Calcium Chloride Dihydrate | 6.00% |
| 4. Thickener (e.g. Xanthan Gum) | 2.00% |
| 5. Preservatives | q.s. |
| 6. Purified Water | q.s. to 100.00% |

### Preparation

In the main vessel add the thickener (e.g. Xanthan Gum) in part of water and disperse thoroughly until homogeneity. Separately solubilize component 2 and component 3 in part of water, previously acidified with component 1. Mix until clear solution is obtained. Combine the two phases adding the acidified solution in the main vessel. Mix under vigorous stirring until to obtain an homogeneous gel. Set the desired pH value with part of component 1.

### EXAMPLE 2

A topical gel formulation, having the following w/w % composition, was prepared:

| | |
|---|---|
| 1. Calcium Trifluoroacetate | 10.00% |
| 2. Thickener (e.g. Hydroxypropyl Cellulose) | 2.00% |
| 3. Preservatives | q.s. |
| 4. Humectants (e.g. PEG-400) | q.s. |
| 5. Purified water | q.s. to 100.00% |

### Preparation

In the main vessel add the thickener (e.g. Xanthan Gum) in part of water and disperse thoroughly until homogeneity. Separately solubilize component 1 in the rest water. Combine the two phases and mix until an homogeneous gel is obtained.

### EXAMPLE 3

An oil in water cream, having the following w/w % composition, was prepared:

| | |
|---|---|
| 1. Calcium Trifuoroacetate | 10.00% |
| 2. Humectant (e.g. Glycerin) | 2.00% |
| 3. Disodium EDTA | 0.10% |
| 4. 5-Ureidohydantoin | 0.05% |
| 5. Panthenol | 0.50% |
| 6. Thickener (e.g. Xanthan Gum) | 0.60% |
| 7. Polyoxyethylene Alkyl Ethers | 5.00% |
| 8. Tocopheryl Acetate | 0.50% |
| 9. Alcohols, C16-18 | 2.00% |
| 10. Stearic Acid | 2.00% |
| 11. Polyoxypropylene Stearyl Ether | 7.00% |
| 12. Bisabolol | 0.10% |
| 13. Preservatives | q.s. |
| 14. Buffers | q.s. |
| 15. Purified water | q.s to 100.00% |

### Preparation

In the main vessel, solubilize components 2, 3, 4, 5 in part of water. Add the thickener and disperse it thoroughly until homogeneity. Then heat the phase at 70 - 75°C. In another vessel combine components 7, 8, 9, 10, 11 and heat the phase at 70 - 75°C while stirring. Combine the two phases heated at the same temperature and homogenize for about 10 minutes. Cool down to 40° and add on sequence components 1 (previously solubilized in part of water), 12 and 13, homogenizing and stirring after each addition.

Cool down to room temperature under moderate stirring and, if necessary, correct the pH value using the proper amount of buffers.

### EXAMPLE 4

An oil in water cream, having the following w/w % composition, was prepared:

| | |
|---|---|
| 1. Calcium Trifluoroacetate | 10.00% |
| 2. Disodium EDTA | 0.10% |
| 3. Panthenol | 1.00% |
| 4. Trimethyl Glycine | 2.00% |
| 5. 5-Ureidohydantoin | 0.20% |
| 6. Propanediol | 5.00% |
| 7. Thickener (e.g. Carbomer) | 0.60% |
| 8. Polyglyceryl-3 Methylglucose Distearate | 3.00% |
| 9. Glyceryl Monostearate | 2.50% |
| 10. Alcohols, C16-18 | 2.50% |
| 11. Heptanoic Acid, 2,2-Dimethyltrimethylene Ester | 4.00% |
| 12. Lactic Acid Dodecyl Ester | 2.50% |
| 13. Serenoa serrulata Fruit Extract, Sesamum indicum Seed Oil, Argania spinosa Kernel Oil, Beta-Sitosterol, Tocopherol | 2.00% |
| 14. Tocopherol, Lecithin, Ascorbyl palmitate, Citric acid | 0.02% |
| 15. Alcohol Denat. | 2.00% |
| 16. Decylene Glycol | 0.50% |
| 17. Benzyl Alcohol | 0.50% |
| 18. Potassium Azeloyl Diglycinate | 3.00% |
| 19. Preservatives | q.s. |
| 20. Corn Starch | 1.00% |
| 21. Purified water | q.s to 100.00% |

### Preparation

In the main vessel, solubilize components 2, 3, 4, 5, part of component 6, in part of water. Add the thickener and disperse it thoroughly until homogeneity. Then heat the phase at 70 - 75°C. In another vessel combine components 8, 9, 10, 11, 12, 13, 14 and heat the phase at 70 - 75°C while stirring. Combine the two phases heated at the same temperature and homogenize for about 10 minutes. Cool down to 40°C while stirring. Separately solubilize components 15, 16, 17 in the remained component 6 and add the phase in the main vessel, stirring for about 5 minutes. Then add on sequence components 18, 19 and 20, homogenizing and stirring after each addition.

Cool down to room temperature under moderate stirring.

The result is an homogeneous cream with a light texture, easy to spread onto the target skin lesions.

### EXAMPLE 5

A topical solution, having the following w/w % composition, was prepared:

| | |
|---|---|
| 1. Sodium Trifluoroacetate | 11.10% |
| 2. Calcium Chloride Dihydrate | 6.00% |
| 3. Trifluoroacetic Acid | 0.14% |
| 4. Hydroxypropyl Chitosan (or Hydroxypropyl Cellulose) | 0.30% |
| 5. Propylene Glycol | 5.00% |
| 6. Purified Water | 35.00% |
| 7. Pvp (Povidone) | 1.00% |
| 8. 2-Propanol | q.s.to 100% |

### Preparation

In the main vessel introduce component 7. Add component 4 (previously solubilized in part of water) and component 5, mix until homogeneity. Separately solubilize components 1, 2, 3 in part of water. Add this solution in the main vessel and mix until to obtain a clear solution.

### EXAMPLE 6

A topical solution, having the following w/w % composition, was prepared:

| | |
|---|---|
| 1. Calcium Trifluoroacetate | 10.00% |
| 2. Salicylic Acid | 2.0% |
| 3. Hydroxypropyl Chitosan (or Hydroxypropyl Cellulose) | 0.50% |
| 4. Dipropylene Glycol | 5.00% |
| 5. Purified Water | 35.00% |
| 6. Buffer | q.s. |
| 7. 2-Propanol | q.s.to 100% |

### Preparation

In the main vessel introduce component 7. Add component 2 and mix until it is completely solubilized. Add on sequence, component 4 and component 1 both previously solubilized in part of water, and component 5. Mix thoroughly until homogeneity.

Adjust pH value with component 6 and mix until to obtain a clear solution.

The two topical solutions described above are quick-drying formulations suitable to be applied on the skin through a brush device or a pump spray. The topical solutions of Example 5 and Example 6 contain film-former polymers (not limited to PVP and HPCH) that contribute in retaining the API ingredient in situ for longer time. Other suitable film-formers can be selected from derivatives of PVP, Chitosan, Cellulose, Acrylates, Polyalkylmethacrylates, Polyesters and also from water-proof polymers such as PVP/Copolymers, Tricotanyl PVP, VP/Polycarbamyl Polyglycol Esters. Furthermore the film former and water-proof effect can be obtained introducing in the topical solutions above described a suitable component of the silicone family, for example Cyclomethicone, Dimethicone, Alkyl Dimethicone, Silica, Silicone Gum, Silicone Elastomers or Resins.

### EXAMPLE 7

A topical water in silicon lotion, having the following w/w % composition, was prepared:

| | |
|---|---|
| 1. Calcium Trifluoroacetate | 10.0% |
| 2. Hydroxypropyl Chitosan | 0.30% |
| 3. Propylene Glycol | 5.00% |
| 4. Purified Water | 41.50% |
| 5. Alkyl Dimethicone Copolyols | 5.20% |
| 6. Cyclomethicone | 35.00% |
| 7. Silicone Resin (e.g. Trimethylsiloxysilicate) | 2.00% |
| 8. Preservatives | q.s. |

### Preparation

In the main vessel introduce components 5, 6, 7 and mix until homogeneity. In separate containers, solubilize component 1 and component 2 in water. Add component 3 in the water phase with component 2. Combine the two water solutions and mix until homogeneity. Add very slowly the water phase in the main vessel while homogenizing with a turbo mixer. Add component 8 and homogenize for few minutes.

The result is a fluid lotion, suitable to be sprayed or applied on the skin with a roll-on device. It contains HPCH and a silicon resin that create an elastic, long lasting and wash-off resistant film on the skin.

### EXAMPLE 8

The exfoliating activity of a formulation containing calcium trifluoroacetate was evaluated *in vivo* after topical application on the back of hairless rats. The formulations used had the following composition: Medicated formulation (D) CaTFA 10.00 g, Xanthan gum 2.00 g, Water q.s. to 100.00 g. Vehicle (V): Xanthan gum 2.00 g, Water q.s. to 100.00 g.

The experiments were carried out on 4 weeks old hairless male rats (HsdHanTM:RNU- Foxn1 rnu, Harlan Italy srl, Correzzana, Italy). The back of ten rats was divided in two parts (section area: 2.5 x 3.0 cm) for topical application of the test formulations. A volume of 200 µl of the medicated formulation or the vehicle was applied two times a day for ten days. Fifteen hours after the last administration, the animals were sacrificed by cervical dislocation and the portion of skin submitted to the treatment was excised, carefully rinsed to remove the remaining formulation and submitted to histological evaluation. A sample of untreated skin of each animal was used as control (C). The histological evaluation was done as follows: the skin samples were fixed in 10% buffered formalin solution, dehydrated and embedded in JB-4 plastic resin. Coronal sections were cut by a microtome (Reichert-Jung) and mounted onto gelatin coated slides. Sections were stained with methylene blue/toluidine blue for microscopic examination to verify the absence of stratum corneum and the condition of the other parts of the skin. The specimens were assessed under Leitz Diaplan microscope.

Results: during treatment some animals showed a mild to severe erythema and some abrasions on the area treated with (D). The results of histological analysis showed the presence of a normal epithelium in all animal areas applied the vehicle, as well as in the untreated control areas. In the areas where the test product D, containing the salt of trifluoroacetic acid, was applied, it was noticed the complete disappearance of the stratum corneum in 6 animals and a significant decrease of thickness in another animal. Only 3 out of the treated animals had no difference between D, V and C. It was concluded that trifluoroacetic acid, calcium salt, was effective as keratolytic agent in 70% of the treated animals.

### EXAMPLE 9

An evaluation of the activity of trifluoroacetic acid was tested on 3 patients (2 females and 1 male) affected by plantar wart (verruca vulgaris), in order to assess the efficacy in terms of disappearance of the skin lesion and pain. The patients, before and during trifluoroacetate treatment, did not take any concomitant treatment with antiviral or keratolytic agents.

The diagnosis of plantar wart was made by clinical examination. Then the subjects were given the composition as per the Example 3. The product was applied twice a day on the skin lesion, and covered by a plastic patch. Duration of treatment lasted 8-15 days.

Clinical evaluation was performed by daily self-assessment of skin thickness, pain at walking and pain at pressure. The results were as follows: pain at walking disappeared in all subjects within 3-7 days. Pain at pressure also disappeared within 7 days. Thickening was reduced starting from the day 7. The man stopped medication at the day 8 after the beginning. The wart was completely disappeared at the day 15. The two women continued the application until the day 15, when the wart spontaneously detached from the underlying skin layers. As a conclusion, the application of a trifluoacetate based cream was successful in treating plantar warts in all subjects. No relapse was noticed in the following weeks.

## Claims

1. Trifluoroacetic acid or a physiologically acceptable salt thereof, for use as keratolytic agent to treat skin or mucosal lesions including actinic (solar) keratosis, common warts and genital warts, follicular hyperkeratinization and acne, plantar hyperkeratosis.

2. Trifluoroacetic acid or a physiologically acceptable salt thereof for use according to claim 1, **characterized in that** it is administered topically.

3. Trifluoroacetic acid or a physiologically acceptable salt thereof for use according to claim 2, **characterized in that** it is administered by means of a semisolid or liquid formulation.

4. Trifluoroacetic acid or a physiologically acceptable salt thereof for use according to claim 3, **characterized in that** said semi-solid or liquid formulation is a solution, an emulsion, a suspension, a cream, a gel or an ointment.

5. Trifluoroacetic acid or a physiologically acceptable salt thereof for use according to claims 3 and 4, **characterized in that** said semi-solid or liquid formulation has a w/w concentration in trifluoroacetic acid or physiologically acceptable salt thereof from 0.1% to 20%, more preferably from 1% to 15%, most preferably from 5% to 10%.

6. Trifluoroacetic acid or a physiologically acceptable salt thereof for use according to any of the preceding claims, **characterized in that** said salt is sodium salt or calcium salt.

7. Trifluoroacetic acid or a physiologically acceptable salt thereof for use according to any of the preceding claims, **characterized in that** it is administered in combination or in temporal proximity with at least one additional active principle.

8. Trifluoroacetic acid or a physiologically acceptable salt thereof for use according to claim 7, **characterized in that** said at least one additional active principle is selected from keratolytic agents; dicarboxylic acid, esters, or amides, plant extracts and their phytosomes, emollients, moisturizers and humectants, plasticizers, anti-oxidants; silicone compounds (siloxanes and silanes); film-forming, suspending and viscosity-increasing polymers; anti-microbials.

9. Trifluoroacetic acid or a physiologically acceptable salt thereof for use according to claim 8, **characterized in that** said at least one keratolytic agent is selected from salicylic acid, glycolic acid, urea, lactic acid, citric acid, malic acid.

10. Trifluoroacetic acid or a physiologically acceptable salt thereof for use according to claim 8, **characterized in that** said at least one dicarboxylic acid, esters, or amides is selected from azelaic acid or potassium azeloyl diglycinate.

11. Trifluoroacetic acid or a physiologically acceptable salt thereof for use according to claim 8, **characterized in that** said at least one plant extracts is selected from Serenoa serrulata Fruit Extract, Sesamum indicum Seed Oil, Argania spinosa kernel oil, bisabolol, trimethylglycine, Boswellia serrata (Olibanum) resin extract, Vaccinium myrtillus seed oil, Oenothera biennis oil, Glycyrrhiza glabra root extract, Epilobium fleischeri extract, Avena sativa extract, Aloe vera extract.

12. Trifluoroacetic acid or a physiologically acceptable salt thereof for use according to claim 8, **characterized in that** said at least one emollient is selected from heptanoic acid, 2,2-dimethyltrimethylene ester, polyoxypropylene alkyl ether, lactic acid dodecyl ester, carbonic acid bis (2-ethylhexyl) ester, essential fatty acids (linoleic and linolenic acids), triglycerides, sterol esters, phytosterols, wax esters, free sterols, free fatty acids, phospholipids, C6-C22 alcohols, n-alkanes, such as paraffinum liquidum, petrolatum, hydrogenated polydecenes.

13. Trifluoroacetic acid or a physiologically acceptable salt thereof for use according to claim 8, **characterized in that** said at least one silicone compound is selected from Cyclomethicone, Dimethicone, Alkyl Dimethicone, Silica, Silicone Gum, Silicone Elastomers or Resins.

14. Trifluoroacetic acid or a physiologically acceptable salt thereof for use according to claim 8, **characterized in that** said at least one moisturizer and/or humectant is selected from polyalcohols, glycerin, sorbitol, propanediol, propylene glycol, dipropylene glycol, polyethylene glycols (PEGs, panthenol; NMF (Natural Moisturizing Factor) components, amino acids, pyrrolidone carboxylic acid and their salts, sodium lactate, peptides.

15. Trifluoroacetic acid or a physiologically acceptable salt thereof for use according to claim 8, **characterized in that** said at least one platicizer is selected from C1-C5 alcohols, urea and ureido compounds, such as allantoin.

16. Trifluoroacetic acid or a physiologically acceptable salt thereof for use according to claim 8, **characterized in that** said at least one anti-oxidant is selected from vitamins and their derivatives,tocopherol, ascorbyl palmitate, tocopheryl acetate.

17. Trifluoroacetic acid or a physiologically acceptable salt thereof for use according to claim 8, **characterized in that** said at least one film-forming, suspending and viscosity-increasing agent is selected from polymers of natural origin like xanthan gum, cellulose derivatives, chitosan derivatives and of synthetic origin, like povidone, copovidone, acrylic derivatives (carbomers), polyamide polymers, alkylamide copolymers.

18. Trifluoroacetic acid or a physiologically acceptable salt thereof for use according to claim 8, **characterized in that** said at least one anti-microbial is selected from decylene glycol, pentylene glycol, benzyl alcohol, phenetyl alcohol, caprylyl glycol, phenylpropanol, ethylhexylglycerin, salicylic acid.
